# EUROPEAN PATENT APPLICATION

(11) **EP 3 379 472 A1**
(43) Date of publication of application: **26.09.2018**
(21) Application number: 17162142.8
(22) Date of filing: 21.03.2017
(51) Int. Cl.: G06Q 10/10, G06Q 50/22

(54) **METHOD AND APPARATUS FOR SENDING A MESSAGE TO A SUBJECT**

(71) Applicant: Koninklijke Philips N.V., 5656 AE Eindhoven (NL)
(72) Inventor: KRANS, Jan Martijn, 5656 AE Eindhoven (NL); BULUT, Murtaza, 5656 AE Eindhoven (NL); DE RUYTER, Boris Emmanuel Rachmund, 5656 AE Eindhoven (NL); VAN DER LANS, Anouk, 5656 AE Eindhoven (NL); CUBA GYLLENSTEN, Illapha Gustav Lars, 5656 AE Eindhoven (NL); VAN DANTZIG, Saskia, 5656 AE Eindhoven (NL)
(74) Representative: de Haan, Poul Erik

(57) **Abstract**

According to an embodiment, there is provided a method of sending a message to a user, the method comprising determining (101) that a message is to be sent to the user; determining (103) a current status of the user; determining (105) the length and/or size of the message based on the current status of the user; generating (107) the message with the determined length and/or size; and sending (109) the message to the user.

## Description

### Technical Field of the Invention

The invention relates to a method and apparatus for sending messages to a user.

### Background to the Invention

In digital coaching applications (apps), users typically receive messages that provide advice, instructions, questions, reminders and/or feedback on their actions via their smartphone or other electronic device. These messages can be provided in the form of notifications or in-app messages. Some of these messages are scheduled at predefined times (and are thus time-based), some messages are delivered in response to the detection of certain events (e.g. that the user has met a behavioural goal). Messages can differ in length (e.g. in terms of the number of characters or number of words, or more generally in terms of the amount and/or type of information conveyed by the message), the format/representation (written text, audio, an image, video, etc.), and complexity. It is appreciated that in view of these variables, some messages take more time, attention and/or mental capacity for a user to read and/or understand than others.

Several factors affect the personal coaching of a user via an application on an electronic device or system of electronic devices. In particular, users can receive multiple or many messages throughout the day, which means that the messages should not unduly interrupt the daily activities of the user. The aim of personal coaching is to achieve a behavioural or habit change in the user, which requires a long term approach and long term usage of the personal coaching application, and thus the personal coaching application should keep the user motivated.

### Summary of the Invention

The attention span or availability of a user during the day to read and/or understand a coaching message can vary a lot. However, the current solutions for the generation and sending of messages to users do not take this into account. For example a user could be sent a message setting out a range of healthy food recipes while the user is performing a physical activity, such as running or attending an exercise class. The user is unlikely to be able to read and understand this long message without interrupting their physical activity. In contrast, when the user is relaxing at home, they will be able to read and understand long and/or complicated messages.

Therefore, the invention provides that the length and/or size of a message is determined based on the current status of the user. This means that the message can be generated with a length and/or size that is appropriate for the current status of the user, increasing the effectiveness of the message.

According to a first aspect, there is provided a method of sending a message to a user, the method comprising determining that a message is to be sent to the user; determining a current status of the user; determining the length and/or size of the message based on the current status of the user; generating the message with the determined length and/or size; and sending the message to the user.

In some embodiments, the current status of the user relates to the ability of the user to read and/or understand a message. The current status of the user can relate to the mental state of the user, the physical state of the user and/or an environmental availability of the user. The physical state of the user may comprise a physical activity of the user and/or a physical activity level of the user. The environmental availability of the user may relate to the location of the user, the weather in the environment of the user and/or a noise level in the environment of the user. The mental state of the user may relate to any one or more of whether the user is asleep, the tiredness of the user, the stress level of the user, the cognitive load of the user, and the affective state of the user.

In some embodiments, the step of determining the length and/or size of the message based on the current status of the user comprises determining the length and/or size of the message based on the ability of the user to read and/or understand the message.

In some embodiments, a plurality of versions of the message are stored in a database, each version having a different length and/or size, and the step of generating the message with the determined length and/or size comprises retrieving the version of the message from the database that has the determined length and/or size, or a length and/or size closest to the determined length.

In alternative embodiments, a default version of the message is stored in a database, the default version of the message having a default length and/or default size, and the step of generating the message with the determined length and/or determined size comprises retrieving the default version of the message from the database; and processing the default version of the message to generate a message with the determined length and/or size if the determined length and/or size of the message is different to the default length and/or size; and otherwise, using the default version of the message as the message to be sent to the user. The step of processing the default version of the message to generate a message with the determined length and/or size comprises removing content from the default version of the message.

The length and/or size of the message corresponds or relates to the amount of information conveyed by the message. The length and/or size of the message can also or alternatively relate to the number of words in the message. The length and/or size of the message can also or alternatively relate to the difficulty of words and/or sentences in the message. The length and/or size of the message can also or alternatively relate to the complexity of ideas/constructs conveyed by the message. The length and/or size of the message can also or alternatively relate to the level of detail of information.

In some embodiments, the method further comprises the step of notifying the user about the reception of the message at an electronic device of the user. In some embodiments, the method can further comprise the step of determining a type of notification to use based on the current status of the user; and the step of notifying the user comprises providing a notification of the determined type.

The message is preferably an electronic message and the step of sending the message to the user preferably comprises sending the electronic message to an electronic device of the user.

In some embodiments, the user has a plurality of electronic devices that can receive messages, and the method further comprises the step of determining which one of the plurality of electronic devices the message is to be sent to is based on the current status of the user and/or based on a format and/or length and/or size of the message, and the step of sending the message to the user comprises sending the message to the determined one of the plurality of electronic devices.

In some embodiments, the step of determining a current status of the user comprises determining the current status from measurements of the user or the activities of the user and/or from information on the user or the activities of the user. In some embodiments, the method further comprises the step of receiving measurements of the user or the activities of the user from one or more sensors, and/or receiving information on the user or the activities of the user from an information source.

According to a second aspect, there is provided a computer program product comprising a computer readable medium having computer readable code embodied therein, the computer readable code being configured such that, on execution by a suitable computer or processor, the computer or processor is caused to perform any of the method embodiments described above.

According to a third aspect, there is provided an apparatus for sending a message to a user, the apparatus comprising a processing unit configured to determine that a message is to be sent to the user; determine a current status of the user; determine the length and/or size of the message based on the current status of the user; generate the message with the determined length and/or size; and send the message to the user.

In some embodiments, the current status of the user relates to the ability of the user to read and/or understand a message. The current status of the user can relate to the mental state of the user, the physical state of the user and/or an environmental availability of the user. The physical state of the user may comprise a physical activity of the user and/or a physical activity level of the user. The environmental availability of the user may relate to the location of the user, the weather in the environment of the user and/or a noise level in the environment of the user. The mental state of the user may relate to any one or more of whether the user is asleep, the tiredness of the user, the stress level of the user, the cognitive load of the user, and the affective state of the user.

In some embodiments, the processing unit is configured to determine the length and/or size of the message based on the current status of the user by determining the length and/or size of the message based on the ability of the user to read and/or understand the message.

In some embodiments, a plurality of versions of the message are stored in a database, each version having a different length and/or size, and the processing unit is configured to generate the message with the determined length and/or size by retrieving the version of the message from the database that has the determined length and/or size, or a length and/or size closest to the determined length.

In alternative embodiments, a default version of the message is stored in a database, the default version of the message having a default length and/or default size, and the processing unit is configured to generate the message with the determined length and/or determined size by retrieving the default version of the message from the database, processing the default version of the message to generate a message with the determined length and/or size if the determined length and/or size of the message is different to the default length and/or size, and otherwise using the default version of the message as the message to be sent to the user. The processing unit can be configured to process the default version of the message to generate a message with the determined length and/or size by removing content from the default version of the message.

The length and/or size of the message corresponds or relates to the amount of information conveyed by the message. The length and/or size of the message can also or alternatively relate to the number of words in the message. The length and/or size of the message can also or alternatively relate to the difficulty of words and/or sentences in the message. The length and/or size of the message can also or alternatively relate to the complexity of ideas/constructs conveyed by the message. The length and/or size of the message can also or alternatively relate to the level of detail of information.

In some embodiments, the processing unit is further configured to notify the user about the reception of the message at an electronic device of the user. In some embodiments, the processing unit is further configured to determine a type of notification to use based on the current status of the user; and the processing unit is configured to notify the user by providing a notification of the determined type.

The message is preferably an electronic message and the processing unit is configured to send the electronic message to an electronic device of the user.

In some embodiments, the user has a plurality of electronic devices that can receive messages, and the processing unit is further configured to determine which one of the plurality of electronic devices the message is to be sent to is based on the current status of the user and/or based on a format and/or length and/or size of the message, and the processing unit is configured to send the message to the user by sending the message to the determined one of the plurality of electronic devices.

In some embodiments, the processing unit is configured to determine a current status of the user from measurements of the user or the activities of the user and/or from information on the user or the activities of the user. In some embodiments, the processing unit is further configured to receive measurements of the user or the activities of the user from one or more sensors, and/or receive information on the user or the activities of the user from an information source.

According to a fourth aspect, there is provided a system comprising an apparatus as described above, wherein the processing unit is configured to determine a current status of the user from measurements of the user or the activities of the user and/or information on the user or the activities of the user; and one or both of: (i) an electronic device for receiving the message from the apparatus, and (ii) one or more sensors for providing the measurements of the user or the activities of the user and/or an information source for providing the information on the user or the activities of the user.

### Brief Description of the Drawings

For a better understanding of the invention, and to show more clearly how it may be carried into effect, reference will now be made, by way of example only, to the accompanying drawings, in which:
Figure 1 is a block diagram of an apparatus as part of a system according to an aspect of the invention; and
Figure 2 is a flow chart illustrating a method of sending a message according to an aspect of the invention.

### Detailed Description of the Preferred Embodiments

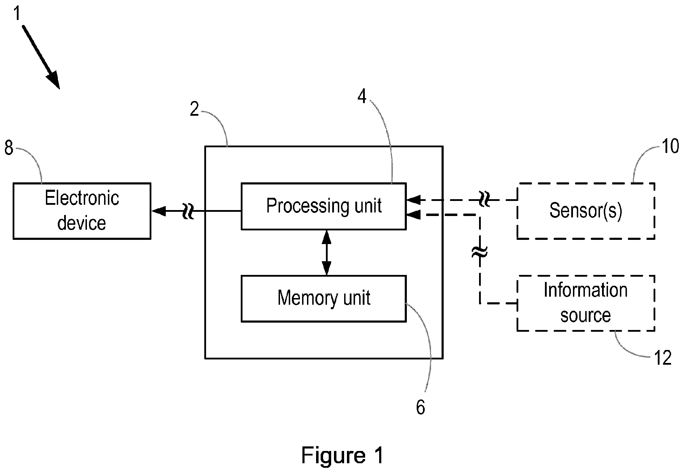

Figure 1 shows a system 1 comprising an apparatus 2 for sending a message to a user according to an embodiment. The apparatus 2 comprises a processing unit 4 and a memory unit 6 that are connected to each other. The processing unit 4 controls the operation of the apparatus 2 and generally implements the method according to the invention. The processing unit 4 can be implemented in numerous ways, with software and/or hardware, to perform the various functions described below. The processing unit 4 may comprise one or more microprocessors or digital signal processor (DSPs) that may be programmed using software or computer program code to perform the required functions and/or to control components of the processing unit 4 to effect the required functions. The processing unit 4 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

In various implementations, the processing unit 4 may be associated with or comprise one or more memory units 6 such as volatile and non-volatile computer memory such as RAM, PROM, EPROM, and EEPROM. The processing unit 4 or associated memory unit 6 can also be used for storing program code that can be executed by a processor in the processing unit 4 to perform the method described herein. In some embodiments, the memory unit 6 can store template messages or content for messages that can be used when generating messages according to the method described below.

Figure 1 also shows the system 1 comprising an electronic device 8 that can be used to present the message to the user. The messages generated according to the invention can be in written format (e.g. text), image format (e.g. pictures or video) audio format, in a haptic format (e.g. a vibration or buzzing) and/or using light, and thus the electronic device 8 can be any type of electronic device 8 that can present a message to a user in one or more of these formats. For example the electronic device 8 can be a smart phone, a tablet, a laptop, a desktop computer, a television (TV), a smart watch, smart glasses/goggles, etc.

In some embodiments, the apparatus 2 is part of the electronic device 8. For example the processing unit 4 can be a central processing unit of a smart phone or tablet. In other embodiments, the apparatus 2 is or is part of a computer that is remote from the electronic devices 8 of the user.

The electronic device 8 is shown as being connected to the processing unit 4/apparatus 2. It should be appreciated that, particularly where the electronic device 8 is separate from the apparatus 2, the electronic device 8 may not be directly connected to the electronic device 8 (e.g. by a wired connection), but the apparatus 2 and the electronic device 8 can be connected in the sense that messages generated by the processing unit 4 can be sent to the electronic device 8 for presentation to the user. Thus, for example, the apparatus 2 can send the message to the electronic device 8 via a direct wireless connection between the apparatus 2 and electronic device 8, e.g. using Bluetooth, Wi-Fi, ZigBee, etc. Alternatively the apparatus 2 can send the message to the electronic device 8 via the Internet, using any suitable wired or wireless connection to the Internet.

Although only a single electronic device 8 is shown in Figure 1, it will be appreciated that the user may have multiple electronic devices 8 that can be used to present messages generated according to the invention.

Where the invention is used as part of a personal coaching application or program, the application or program could be executed or run by the processing unit 4. In some embodiments, the personal coaching application or program can be installed or run of each of several electronic devices 8 of the user.

As described in more detail below, the current status of the user is determined, and the processing unit 4 can determine the current status based on measurements of the user or their activities, or information on the user or their activities. Therefore, the apparatus 2 can be configured to receive measurements from one or more sensors 10 and/or information from one or more information sources 12. Further details of possible types of the sensors 10 and information sources 12 that can be used are set out below with reference to specific embodiments.

As with the electronic device 8, although the sensor(s) 10 and information source(s) 12 are shown as being connected to the processing unit 4/apparatus 2. It should be appreciated that the sensor(s) 10 and information source(s) 12 may not be directly connected to the apparatus 2 (e.g. by a wired connection), but the apparatus 2 and the sensor(s) 10 and information source(s) 12 can be connected in the sense that measurements by the sensor(s) 10 and information from the information source(s) 12 can be sent to the apparatus 2. Thus, for example, the apparatus 2 and sensor(s) 10 and information source(s) 12 can be connected via a direct wireless connection, e.g. using Bluetooth, Wi-Fi, ZigBee, etc, or alternatively they can be connected via the Internet, using any suitable wired or wireless connection to the Internet.

As noted above, the invention provides that the length and/or size of a message that is to be sent to the user is determined based on the current status of the user. This means that the message can be generated with a length and/or size that is appropriate for the current status of the user, increasing the effectiveness of the message in the sense that the user is able to read and/or understand the message.

Briefly, when a message is to be sent to the user, the current status of the user is determined, which can be in terms of the physical state of the user, the mental state of the user, the environmental conditions or environmental state around the user (all of which can be termed the 'availability' of the user). Then the message is generated having an appropriate length and/or size (which is also referred to as content density) for the availability of the user. In some embodiments, different versions of a message that have respective lengths/sizes/content density levels can be stored in a memory unit 6 or database, the generation of the message can involve retrieving the appropriate version of the message from the memory or database (also referred to as a 'library'). In other embodiments, a default version of a message can be stored in a memory or database and a message having the required length/size/content density level can be generated from that default version. The message can then be sent to the user, for example via an electronic device 8 of the user.

Thus, in some embodiments, a library of messages is provided that contains multiple versions of each message. That is, for each message, several versions with different length, size, content density and/or information load are provided. In some embodiments, versions of the messages can be provided that are in different formats, for example written text, pictures or video or audio.

As an example, in the case of a text-based message, versions of the message having different lengths could be available in the library: e.g. short, medium and long versions. At some moments during the day a user may have time for reflection and can read longer text messages that for example contain more background information than shorter versions of the message. However, at other moments the user may be busy and could benefit from a short or shorter message containing advice, a question, a reminder or a reward, since they do not have time to read a long message.

Since users typically have several connected electronic devices 8, there may be a number of different devices 8 that the message could be sent to and used to present the message to the user. For example at different times of the day a user may consume content from different information-providing devices, such as a smartphone, a personal computer (PC), a tablet, a TV, a bathroom mirror display, a speaker, etc., and at a given moment during the day the user's attention is predominantly directed to only one or two of these devices. Delivering a message to a device 8 that the user is not using may mean that the message is missed, or is not as effective as it should be once it is received by the user. Therefore in some embodiments, the message can be sent to all of the user's electronic devices 8. However this could result in the message 'popping up' or being notified to the user on several different devices 8 at once, which could be distracting or annoying. Therefore, in more preferred embodiments, when a message is to be sent to the user, the availability of a certain electronic device 8 or the availability of any of the electronic devices 8 belonging to the user can be determined, and the message can be sent to an appropriate electronic device 8 for presentation to the user. In these embodiments, the availability of the device 8 refers to the availability of the device 8 to the user, and can include identifying the electronic device 8 that is closest to the user in terms of distance or location (e.g. in the same room, etc.), that was last used by the user, and/or that is currently being used by the user. These embodiments are referred to herein as selecting or adjusting the 'delivery channel' of the message, i.e. the channel by which the message is delivered to the user.

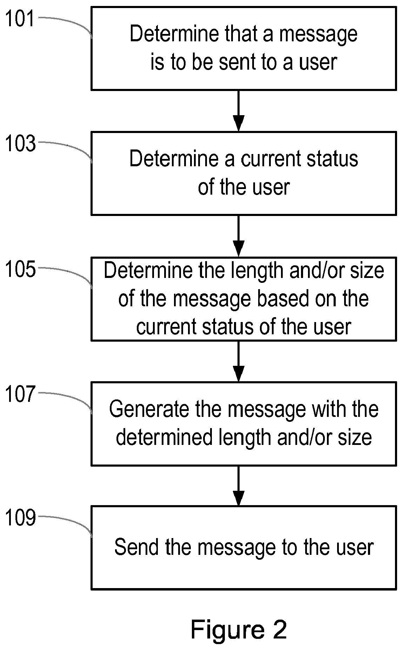

Methods of generating and sending messages according to the invention are described in more detail below with reference to Figure 2. Figure 2 is a flow chart illustrating various steps in the method according to the invention. It will be appreciated that many, if not all, of the method steps can be performed by the apparatus 2, particularly the processing unit 4.

In a first step, step 101, it is determined that a message is to be sent to the user. A message may be sent to the user for a variety of reasons, for example to provide advice, questions, instructions, reminders and/or feedback on their actions. The timing of the sending of the messages may depend on a message schedule or be in response to certain actions (or inaction) by the user. The need to send a message at a particular time can be determined by an application or program, such as a personal coaching application. Those skilled in the art will be aware of various ways in which the need to send a message can be determined, and thus further details are not provided herein.

It will be appreciated that step 101 may include determining the purpose of the message. That is, the type of information that is to be conveyed by the message may be determined in step 101. For example, in step 101 it may be determined that a message should be sent to the user to remind them to eat a healthy meal. In that case the type of information to be conveyed by the message is a reminder to eat a healthy meal. As another example in step 101 it may be determined that a message should be sent to the user to encourage them to attend an exercise class. In that case the type of information to be conveyed by the message may encourage the user to attend an exercise class, and/or provide information on nearby exercise classes.

In step 103, a current status of the user is determined. As noted above, the current status of the user can be in terms of the current physical state of the user, the current mental state of the user, the current environmental conditions or current environmental state around the user. In general, the current status of the user relates to the ability of the user to read and/or understand a message.

The physical state of the user can relate to the current physical activity of the user and/or a physical activity level of the user and can indicate the availability of the user to read and/or understand a message. The physical state can be determined, for example, from measurements by a movement or activity sensor 10 (such as an accelerometer, gyroscope, etc.) that is worn or carried by the user. The physical activity can be determined in terms of particular types of activity, such as walking, sitting down, running, exercising, cycling, sleeping, etc. A physical activity level can comprise a measure of the activity level of the user on a determined scale, e.g. low activity level (which could correspond to, e.g., the user sitting down and being idle), medium activity level (which could correspond to, e.g., the user walking), and high activity level (which could correspond to, e.g., the user running or cycling). Those skilled in the art will be aware of various ways in which a physical state or physical activity level of a user can be determined.

The mental state of the user can relate to the mental capacity of the user to read and/or understand a message. For example the mental state can relate to any one or more of whether the user is asleep, the level of tiredness of the user, the stress level of the user, the cognitive load of the user, and the affective state of the user.

The mental state of the user can be determined, for example from measurements by a movement or activity sensor 10 (such as an accelerometer, gyroscope, etc.) that is worn or carried by the user. A movement or activity sensor 10 can, for example, be used to detect whether the user is sleeping or laying down.

Another or alternative sensor 10 that can be used is a microphone or other sound sensor for monitoring the sounds made by the user (including speech by the user) and/or in the environment of the user. An analysis of the speech of the user, for example in terms of the tone or volume of the user's voice, or the content of the speech (e.g. the words spoken), can be used to estimate the mental state of the user. Those skilled in the art will be aware of various techniques and algorithms for processing sound measurements to identify speech by the user and to analyse the speech to estimate or determine the mental state of a user. Therefore further details of this processing are not provided herein. Other relevant sounds that can be measured by a microphone or sound sensor include the user yawning, the breathing rate of the user, the heart rate, etc.

Another or alternative sensor 10 that can be used is an electroencephalogram (EEG) sensor that can measure electrical activity in the brain. Such a sensor could be integrated into a headset or other head-worn device. The electrical activity in the brain can be indicative of the mental state of the user (including the sleep state of the user), and thus the mental state of the user can be determined or estimated from an analysis or processing of the EEG measurements. Those skilled in the art will be aware of various techniques and algorithms for processing EEG measurements to estimate or determine the mental state of a user and thus further details are not provided herein.

Another or alternative sensor 10 that can be used is a sensor that can measure one or more physiological characteristics of the user, such as heart rate, heart rate variability, galvanic skin resistance, skin temperature, blood pressure, breathing rate, etc. It is known that certain mental states can result in a measurable response or change in one or more of these physiological characteristics and thus the mental state of the user can be estimated or determined based on an evaluation of the physiological characteristics. Those skilled in the art will be aware of various techniques and algorithms for processing physiological characteristic measurements to estimate or determine the mental state of a user and thus further details are not provided herein.

The mental state of the user can also or alternatively be determined or estimated based on analysing recent events or activities by the user (e.g. the user was reading a newspaper or using an electronic device, how well the user slept the night before, how busy the user was at work that day), the duration and pressure used in touch operations on a touch screen or button(s) (e.g. on a keyboard) of an electronic device 8, the level of self-corrections required by a user (and then effected by the user) when using an electronic device 8 (e.g. the level of typing errors and the proportion of these errors that are then corrected by the subject), and/or the facial expression of the user.

The environmental conditions or environmental state of the user can relate to the location of the user, the weather in the environment of the user and/or a noise level in the environment. The location of the user can indicate, for example, whether the user is in a vehicle, whether the user is at home, at work, at the shops, gym, etc., which room of the home the user in, etc. The location of the user can be measured by a sensor 10 that is worn or carried by the user (e.g. a satellite positioning system (e.g. GPS) receiver, or a radio frequency (RF) signal receiver that can receive signals, such as Wi-Fi or cellular signals, from which the location of the user can be inferred or determined), for example that is part of an electronic device 8 of the user, such as a smart phone. In addition or alternatively, the location of the user can be estimated from events contained in a calendar or schedule for the user. This calendar or schedule (i.e. an information source 12) can contain information on where the user is expected to be (e.g. at work, in a meeting, at a party, etc.).

The weather in the environment can be determined by looking up the current location of the user in a weather database 12, for example that is accessible via the Internet. The weather information can indicate, for example, whether it is sunny, cloudy, raining, foggy, etc.

The environmental conditions or state can also or alternatively include the noise level in the environment of the user, which can be measured using a microphone or other sound sensor 10. In another example, the environmental conditions can indicate the level of pollution (i.e. the amount of particles in the air). This could be measured by a dedicated pollution sensor, or by a sensor in an air purifier. In another example, the environmental conditions indicate the level of carbon dioxide CO₂ in the air.

In step 103, one or more of the above types of information can be determined and combined to determine the current status of the user. In some embodiments, a score can be assigned to each element of information that is determined, and these scores combined to form an overall status score for the user. For example a score can be associated with the determined physical activity of the user, a score can be associated with the determined mental state of the user (or respective scores can be determined for different aspects of the mental state of the user), a score can be associated with the determined environmental conditions (or respective scores can be determined for different aspects of the environmental conditions), and these scores combined in any suitable way (e.g. addition, multiplication, division, subtraction, or any combination) to determine an overall status score. In some embodiments, the overall score can be in the range of 0 to 1, or any other normalised or predetermined range, where a value at one end of the scale (e.g. 0) can represent that the current status of the user is that they are not available to read and/or understand a message (or read and/or understand a long message), and a value at the other end of the scale (e.g. 1) can represent that the current status of the user is that they are available to read and/or understand a message (or read and/or understand a long message). In alternative embodiments, rather than assign a score to each element of information, a data mining approach can be used where an overall score is derived directly from the different types of information.

It will be appreciated that in some embodiments steps 101 and 103 are performed sequentially, but in other embodiments steps 101 and 103 can be performed at the same time, or step 103 can be performed continuously or regularly in order for the method/apparatus 2 to be aware of the current status of the user at all times, so that a current status is available when it is determined in step 101 that a message is to be sent to the user.

Next, in step 105, the length and/or size of the message is determined based on the current status of the user determined in step 103. Thus, the length and/or size of the message is determined based on the ability of the user to read and/or understand the message. The length and/or size of the message affects the amount of information and level of detail conveyed by the message, which can relate to the number of words in the message (whether written or spoken), the level of difficulty required to understand the words or sentences in the message (where the difficulty can relate to how frequently a particular word, phrase, or sentence has been used by the user or other people), and/or the complexity of ideas or concepts conveyed by the message and the level of detail of the information. Where the message is a question, the length and/or size of the question is determined based on the current status of the user, and this can also include determining the ease with which the question can be answered, e.g. whether a long written answer is required, whether the question is presented as multiple choice, yes/no, etc.

In some embodiments, a number of different versions of a message are stored in a database (e.g. in memory unit 6), with each version having a different length and/or size. In this case, step 105 can comprise determining which version of the message is required based on the current status.

Table 1 below illustrates an embodiment of step 105 in which three versions of each message are provided. Thus there are three messages, Message A, Message B and Message C, each with small, medium and large versions relating to the length and/or size of the message. Each version is associated with a range of possible scores for the current status of the user. In this embodiment the status is a score in the range of 0 to 1, where a higher score indicates a higher availability of the user to read and/or understand a message.

**Table 1**

| | **0<Score<0**.**3** | **0.3<Score<0.6** | **0.6<Score<1.0** |
|---|---|---|---|
| **Message A** | Message A Small | Message A Medium | Message A Large |
| **Message B** | Message B Small | Message B Medium | Message B Large |
| **Message C** | Message C Small | Message C Medium | Message C Large |
| ***Message ...*** | | | |

Thus, for a status score in the range of 0.3 to 0.6, the medium version of the required message (e.g. Message B) is selected, whereas the small version is selected for status scores below this range and the large version is selected for status scores above this range.

In a second embodiment, a default version of the message is stored in a database (e.g. in memory unit 6), with the default version of the message having a default length and/or default size. In this case, step 105 comprises determining whether to use the default version of the message, or whether a shorter or longer version of the message is required.

In a third embodiment, step 105 can comprise determining a desired length and/or size from the current status determined in step 103, without reference to specific versions of a message stored in a database 8. It will be appreciated that in this embodiment an 'optimum' length and/or size of the message can be determined that is optimal for the current status of the user.

Having determined the length and/or size of the message in step 105, in step 107 the message is generated having the determined length and/or size.

In the first embodiment above where multiple versions of a message are stored in a library or database 6, step 107 comprises retrieving the version of the message from the database 6 that has the determined length and/or size.

In the second embodiment above where a default version of a message is stored in a database 6, step 107 can comprise retrieving the default version of the message from the database 6 and processing the default version of the message to generate a message with the determined length and/or size if the required length and/or size of the message determined in step 105 is different to the default length and/or size. If the required length and/or size of the message determined in step 105 is the same as the default length and/or size, then the default message can be used as message to be sent to the user.

Processing the default version of the message to generate a message with the determined length and/or size can comprise removing or adding content from the default version of the message. Changing the content can mean adding or removing one or more words or sentences, adding or removing certain information, e.g. information considered to be background information to the main content of the message, adding or removing pictures or video, changing the complexity of the content of the message, e.g. using words that are easier and/or quicker to understand, etc. Methods and techniques for processing messages to alter their content are known to those skilled in the art, so further details are not provided herein.

As an example, a default version of a message stored in a database 6 can correspond to a 'master' text for a message, i.e. it is the full and largest version of the message. Where a status score is derived as described above, and in an example where the score is provided on a scale of 0 to 1 where a higher score indicates a higher availability of the user to read and/or understand a message, the default version of the message can be used as the message to be sent in the case where the status score is 1. Where the status score is less than 1, words and/or sentences can be removed from the master text in order to reduce the size and/or length of the generated message. Natural language processing algorithms can be used for this. In some embodiments, the amount of words removed from the text can be inversely proportional to the status score. For example, if the status score is 0.5, then 50% of the words are removed, and if the status score is 0.1, then 90% of the words are removed. It will be appreciated that this is just an example, and a different relationship between the status score and the amount of words to be removed can be used.

In the third embodiment above where an optimal length and/or size for the message is determined in step 105, and where there are multiple versions of each message stored in a database 6 as in the first embodiment, step 107 can comprise retrieving the version of the message having the length and/or size closest to the determined length and/or size.

In a variation of the third embodiment, which is a combination of the above first and second embodiments, where an optimal length and/or size for the message is determined in step 105 and where there are multiple versions of each message stored in a database 6 as in the first embodiment, step 107 can comprise retrieving the version of the message having the length and/or size closest to the determined length and/or size, and processing the retrieved message to add or remove content to adjust it to the determined length and/or size.

Once the message has been generated in step 107, the message is sent to the user in step 109. It will be appreciated that as the message is generated based on the current status of the user, the message is preferably sent to the user as soon as possible, i.e. while the message is still applicable to the status of the user and before the 'current' status of the user changes. This step can comprise sending the electronic message to an electronic device 8 of the user. Where the processing unit 4/apparatus 2 is part of an electronic device 8 of the user, step 109 can comprise displaying or otherwise presenting the message to the user on a user interface of the electronic device 8 (e.g. a display screen or speaker). Where the processing unit 4/apparatus 2 is separate from an electronic device 8 of the user (or separate from the electronic device 8 that the message is to be sent to, step 109 can comprise sending the message to that electronic device 8, e.g. via a wireless connection, and that electronic device 8 displaying or otherwise presenting the message to the user on a user interface of the electronic device 8 (e.g. a display screen or speaker).

It will be appreciated that step 109 can comprise sending the message to the user, e.g. to an electronic device 8 of the user so that it is available for the user to view/hear, and the electronic device 8 providing a notification to the user that a message is available. The notification can be in the form of a pop-up message, a display icon and/or an audible sound or tone. The notification may be a push notification, i.e. a notification that is pushed to the electronic device 8 from the apparatus 2.

In some embodiments, where a notification is provided to the user by an electronic device 8 that the message is available to view/hear, the method can further comprise determining a type of notification to use based on the current status of the user then providing a notification of the determined type. Types of notification include a pop-up message or icon on a display screen, playing an audible sound or tone and/or vibrating or buzzing. It will be appreciated that a notification can be considered as a type of message, and thus the steps performed in steps 105 and 107 with respect to the message to be sent to the user can be applied equally to determining a suitable type of notification to use according to the current status of the user.

Table 2 below illustrates an embodiment in which the type of notification to use is determined based on the current status of the user. There are three types of notification, a buzz (vibration), a sound and a light (flash). The possible status scores for the current status of the user are divided into three categories, the same as in Table 1. In this embodiment the status is a score in the range of 0 to 1, where a higher score indicates a higher availability of the user to read and/or understand a message.

**Table 2**

| | **0<Score<0**.**3** | **0.3<Score<0.6** | **0.6<Score<1.0** |
|---|---|---|---|
| **Buzz** | √ | √ | √ |
| **Sound** | √ | √ | |
| **Flash** | √ | | |

Thus in this example, when the user is least available to receive a message, all three types of notification are used to notify the user that there is a message available. When the user is most available to receive a message, in this example only the vibration notification is used.

As noted above, a user can have multiple electronic devices 8 (and multiple different types of electronic devices 8) that can be used to receive and present messages. In some embodiments, step 109 can comprise sending the message to one of the electronic devices 8 of the user, for example a default device 8 for the user, or a device 8 that the user has selected as being preferred for receiving messages. In other embodiments, step 109 can comprise sending the message to all of the electronic devices 8 of the user.

However, in preferred embodiments, before the message is sent to the user in step 109, the method can comprise determining which one of the number of electronic devices 8 the message is to be sent to based on the current status of the user, and then step 109 comprises sending the message to the determined electronic device. The electronic device 8 to be used can be the electronic device 8 that is closest to the user in terms of distance or location (e.g. in the same room, etc.), the electronic device 8 that was last used by the user and/or that is currently being used by the user. Where the user is using multiple devices 8, e.g. watching the TV and using their smart phone, the message could be sent to either device 8, or the system 1 could further comprise means to determine which device 8 the subject is focussed on at that time and the message can be sent to that device 8. Alternatively, in the above scenario, part of the message can be displayed on the TV and part can be displayed on the smart phone.

Table 3 below illustrates an exemplary mapping between certain statuses of the user and the electronic device 8 that the message is to be sent to. In particular, the user has several electronic devices 8 including a smart phone, a laptop, a tablet, a TV, a smart watch and a dashboard display in a vehicle, and Table 3 shows the devices 8 in an order of preference based on the status of the user (shown in the top line). The most preferred device 8 is shown in the second line of the table and the least preferred device 8 is shown in the fourth line.

**Table 3**

| **Car driving** | **On a train** | **Riding a bike** | **Running** | **On couch/sofa** | **In a meeting** |
|---|---|---|---|---|---|
| Dashboard | Laptop | Dashboard | Watch | TV | Laptop |
| Watch | Tablet | Watch | Phone | Tablet | Phone |
| (Phone) | Phone | (Phone) | | Phone | |

In further embodiments, the electronic device 8 can be further determined or identified based on a format and/or length and/or size of the message. For example it may not be appropriate to send a long/large message to a smart watch, in which case a device 8 with a larger screen could be selected if there are two or more electronic devices 8 that could be used to receive the message based on the current status of the user.

In further embodiments, the format of the message (i.e. written text, audio message, video) can be determined based on the current status of the user. For example if the current status of the user indicates that the user is driving then it is more appropriate for the message to be provided in an audio format than a written or video format. Similarly, if the user is in a noisy environment, it may be better to provide the message in a written format rather than an audio format.

As noted above, the invention aims to optimise the message content and delivery medium so that it will have the highest effectiveness. The message effectiveness is an objective measure that can be calculated from the user's response to the message and other data. This can be measured as follows. Initially the message content length/size and delivery medium can be determined as described above. Since in this beginning stage there may not be sufficient user data, heuristics-based algorithms can be used. Each time a message is sent to the user, the user is monitored to determine how well the user has followed the message. This is the objective measure mentioned above. The objective measure for each message may require different user aspects to be monitored. For example, messages that encourage or require the user to perform high activity will require the user's activity to be monitored, while messages relating to stress coaching or dealing with stress may require monitoring of heart rate variability (which varies with stress level) and perhaps some additional user input through questionnaires.

It will also be appreciated that the apparatus 2 can analyse the behaviour and responsiveness of the user over time and further adapt the message length/size and selected delivery channel to optimise the user's response to the message. For example it may be found that a user never responds or adapts their behaviour when a message is sent while they are running. In that case, if the current status is determined in step 103 to be running, then in that case a message may not be sent until the user has stopped running.

In some embodiments, where a message with a short size and/or length is sent to the user, the user can be presented with that version of the message, along with an option to receive a larger or full version of the message. In that case the apparatus 2 sends a larger or the full version of the message to the user.

There is therefore provided an improved method and apparatus for sending messages to a user.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method of sending a message to a user, the method comprising:
determining (101) that a message is to be sent to the user;
determining (103) a current status of the user;
determining (105) the length and/or size of the message based on the current status of the user;
generating (107) the message with the determined length and/or size; and
sending (109) the message to the user.

2. A method as claimed in claim 1, wherein the current status of the user relates to the ability of the user to read and/or understand a message.

3. A method as claimed in claim 1 or 2, wherein the current status of the user relates to the mental state of the user, the physical state of the user and/or an environmental availability of the user.

4. A method as claimed in any of claims 1-3, wherein a plurality of versions of the message are stored in a database, each version having a different length and/or size, and wherein the step of generating (107) the message with the determined length and/or size comprises:
retrieving the version of the message from the database that has the determined length and/or size, or a length and/or size closest to the determined length.

5. A method as claimed in any of claims 1-3, wherein a default version of the message is stored in a database, the default version of the message having a default length and/or default size, and wherein the step of generating (107) the message with the determined length and/or determined size comprises:
retrieving the default version of the message from the database; and
if the determined length and/or size of the message is different to the default length and/or size, processing the default version of the message to generate a message with the determined length and/or size;
otherwise, using the default version of the message as the message to be sent to the user.

6. A method as claimed in any of claims 1-5, wherein the method further comprises the steps of:
determining a type of notification to use to notify the user about the reception of a message based on the current status of the user; and
providing a notification of the determined type to the user.

7. A method as claimed in any of claims 1-6, wherein the message is an electronic message and the step of sending the message to the user comprises sending the electronic message to an electronic device of the user.

8. A method as claimed in claim 7, wherein the user has a plurality of electronic devices that can receive messages, and wherein the method further comprises the step of:
determining which one of the plurality of electronic devices the message is to be sent to is based on the current status of the user and/or based on a format and/or length and/or size of the message; and
wherein the step of sending the message to the user comprises sending the message to the determined one of the plurality of electronic devices.

9. A computer program product comprising a computer readable medium having computer readable code embodied therein, the computer readable code being configured such that, on execution by a suitable computer or processor, the computer or processor is caused to perform the method of any of claims 1-8.

10. An apparatus (2) for sending a message to a user, the apparatus (2) comprising a processing unit (4) configured to determine that a message is to be sent to the user; determine a current status of the user; determine the length and/or size of the message based on the current status of the user; generate the message with the determined length and/or size; and send the message to the user.

11. An apparatus (2) as claimed in claim 10, wherein a plurality of versions of the message are stored in a database (6), each version having a different length and/or size, and wherein the processing unit (4) is configured to generate the message with the determined length and/or size by:
retrieving the version of the message from the database (6) that has the determined length and/or size, or a length and/or size closest to the determined length.

12. An apparatus (2) as claimed in claim 10, wherein a default version of the message is stored in a database (6), the default version of the message having a default length and/or default size, and wherein the processing unit (4) is configured to generate the message with the determined length and/or determined size by:
retrieving the default version of the message from the database (6); and
if the determined length and/or size of the message is different to the default length and/or size, processing the default version of the message to generate a message with the determined length and/or size;
otherwise, using the default version of the message as the message to be sent to the user.

13. An apparatus (2) as claimed in any of claims 10-12, wherein the processing unit (4) is further configured to:
determine a type of notification to use to notify the user about the reception of a message based on the current status of the user; and
provide a notification of the determined type to the user.

14. An apparatus (2) as claimed in any of claims 10-13, wherein the message is an electronic message and the processing unit (4) is configured to send the electronic message to an electronic device (8) of the user.

15. A system (1) comprising:
an apparatus (2) as claimed in any of claims 10-14, wherein the processing unit (4) is configured to determine a current status of the user from measurements of the user or the activities of the user and/or information on the user or the activities of the user; and
one or both of: (i) an electronic device (8) for receiving the message from the apparatus (2), and (ii) one or more sensors (10) for providing the measurements of the user or the activities of the user and/or an information source (12) for providing the information on the user or the activities of the user.
